(19) Europäisches Patentamt
European Patent Office
Office européen des brevets

(11) Publication number: **0 141 569**
**B1**

# (12) EUROPEAN PATENT SPECIFICATION

(45) Date of publication of patent specification: **21.12.88**

(21) Application number: **84307057.4**

(22) Date of filing: **15.10.84**

(51) Int. Cl.[4]: **C 07 C 49/203,** C 07 C 45/74, C 07 C 45/72, C 07 C 45/66, A 61 K 7/46, C 11 B 9/00

## (54) Perfumery compounds and their preparation.

(30) Priority: **25.10.83 GB 8328480**

(43) Date of publication of application:
**15.05.85 Bulletin 85/20**

(45) Publication of the grant of the patent:
**21.12.88 Bulletin 88/51**

(84) Designated Contracting States:
**AT BE CH DE FR GB IT LI NL**

(56) References cited:
**GB-A-1 059 839**

**JOURNAL OF THE AMERICAN CHEMICAL SOCIETY, vol. 66, March 1944, pages 372-376;**

**S.G. POWELL et al.: "The condensation of isobutyraldehyde with aliphatic ketones" CHEMICAL ABSTRACTS, vol. 90, 1979, page 359, no. 12161z, Columbus, Ohio, US; I.A. ASTAKHOVA et al.: "Preparation of 2,7-dimethyloctan-2-ol from methylisobutylketone and isobutyraldehyde" & MASLO-ZHIR. PROM-ST. 1978, (9), 26-7**

(73) Proprietor: **BUSH BOAKE ALLEN Limited**
**Blackhorse Lane Walthamstow**
**London E17 5QP (GB)**

(72) Inventor: **Ansari, Hifzur Rahman**
**7 Mercedes Drive**
**Montvale New Jersey 07645 (US)**
Inventor: **Webb, David**
**Blackhorse Lane**
**Walthamstow London E17 5QP (GB)**
Inventor: **Schleppnik, Alfred A.**
**One Hanley Downs**
**St. Louis Missouri 63117 (US)**

(74) Representative: **Lawrence, Peter Robin Broughton et al**
**GILL JENNINGS & EVERY 53-64 Chancery Lane**
**London WC2A 1HN (GB)**

(56) References cited:
**ANGEWANDTE CHEMIE, INTERNATIONAL EDITION IN ENGLISH, vol. 17, no. 6, 1978, pages 450-451; R. LOHMAR et al.: "Alpha-amino acids as nucleophilic acyl equivalents: synthesis of ketones and aldehydes by means of oxazolin-5-ones"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 141 569 B1**

Courier Press, Leamington Spa, England.

(56) References cited:

**BULLETIN DE LA SOCIETE CHIMIQUE DE FRANCE, nos. 9-10, 1977, pages 925-930; B. CAZES et al.: "Carbanions lithiés d'alpha'-cyanoéthers beta,gamma-insaturés. I. Réarrangements sigmatropiques 2.3r en série allylique; préparation de cétones beta,gamma-éthyléniques"**

**CHEMICAL ABSTRACTS, vol. 92, no. 13, March 1980, page 614, no. 110486v, Columbus, Ohio, US; S. WATANABE et al.: "Syntheses of allylketones, alpha,beta-unsaturated ketones, and methylketones from carboxylic acids" & YUKAGAKU 1979, 28(11), 862-6**

## Description

The great majority of synthetic perfumery compounds have a terpenoid or other cylic structure and there are relatively few synthetic perfumery compounds having a substantially linear structure.

If a compound is to serve as a synthetic perfumery compound it has to comply with three essential requirements and failure of either requirement will prevent it being a useful synthetic perfumery compound. One requirement is that the compound has, at low concentrations, a pleasant odour and can be blended with other perfumery compounds to give pleasant blended odours. Another is that it is stable in compositions in which it can be used, for instance when used, in, e.g., soap or shampoo its odour and other properties must not change on storage. The other requirements is that the compound must be capable of being synthesised at low cost from readily available starting materials. There is little or no commercial interest in compounds as synthetic perfumery compounds if their synthesis requires expensive and poorly available starting materials or if it requires expensive process steps, since the reason for providing synthetic perfumery compounds is to get away from the expense of natural perfumery compositions.

Most of the thousands or millions of low molecular weight aliphatic compounds have an odor but, despite this, very few of them are useful as synthetic perfumes since very few have the required combination of useful odour characteristics (especially when blended), stability to compositions in which they can be used (e.g., soaps), and low cost.

A few compounds having a chain of 8 carbon atoms are known to have pleasant odours, for instance as disclosed in "Perfume and Flavour Materials", Volumes I and II, by S. Arctander (published 1969). One such compound is generally known as dimethylocteneone and in particular is 4,7 - dimethyl - 6 - octene - 4 - one (GB 1,059,839, compound 62). However this compound is very costly to make and has a green geranium, oil odour.

In GB 1,059,839 Example 64 shows a mixture of 2,7 - dimethyl - 6 - octen - 3 - one and 3,3,6 - trimethyl - 5 - chepten - 2 - one.

Another octeneone compound is 2,6 - dimethyl - octa - 5,7 - diene - 4 - one. This occurs naturally in tagetes but is not available commercially. It is known to have an odour but it has not previously been proposed as contributing useful odour properties to perfumery compositions and, in particular, it has been very expensive and difficult to make. Accordingly it has not been adopted as a commercially useful synthetic perfumery compound.

In the invention the compound 2,7 - dimethyl - oct - 5 - ene - 4 - one is used as a perfumery component in a perfumery composition comprising the dimethylocteneone compound and at least one other odiferous chemical. The compound 2,7 - dimethyl - oct - 5 - ene - 4 - one is alternatively known as 2,7 - dimethyl - oct - 3 - ene - 5 - one and has the formula

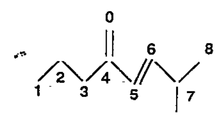

The 5-ene compound may be used free of the 6-ene and 7-ene isomers or blended with either or both of these isomers. Preferred blends that may be used in the invention may contain at least 20%, preferably at least 50% and most preferably at least 75% by weight of the 5-ene compound with the balance preferably being the 6-ene compound or, less preferably, a mixture of the 6-ene compound with a small amount, typically 1 part in 6, of the 7-ene compound.

The compound used in the invention, is of particular value when used in synthetic perfumery compositions because of the combination of perfumery properties and ease of synthesis.

A perfumery composition generally comprises a large number of odiferous chemicals. The 5-ene compound may be provided as a concentrate for incorporating in a consumer composition or may be provided as a consumer composition. The composition can be one of a wide variety of consume compositions, but preferably take the form of a cologne, cream, lotion, soap, detergent, air freshener or antiperspirant. Particularly preferred compositions are colognes and especially, air fresheners, for instance aerosol air fresheners.

The 5-ene compound used in the invention, has a very strong odour having fruity, taget and chamomile characteristics. It is of particular value for contributing a natural jasmine odour and, especially of imparting a natural note to Bergamot and lemon fragranges.

The compound used in the invention is of suitable stability for most perfumery uses, but the compositions in which it is formulated are preferably not highly alkaline or highly acidic. Preferably the compositions have pH between 3 and 10, generally between 5 and 8.

The invention includes a process for making a perfumery composition comprising making 2.7 dimethyl - oct - 5 - ene - 4 - one by reacting 2 - methyl - pentan - 4 - one with isobutyraldehyde and dehydrating the resultant saturated product and then combining the resultant 2,7 - dimethyl - oct - 5 - ene - 4 - one with at least one odiferous compound that is not a dimethyl octerone.

The process may be conducted in the presence of a mixed solvent such as aqueous dioxane (in the presence of alkali) but preferably is conducted as a heterogeneous mixture of aqueous alkali, ketone and aldehyde. To promote transfer between the phases the reaction is preferably conducted in the presence of a phase transfer catalyst. A suitable catalyst is cetyl trimethylammonium bromide but other phase

transfer catalyst that may be used include other tetra alkyl quaternary ammonium halides such as tetrabutyl ammonium iodide and tricaprylyl methyl ammonium chloride. The ratio by weight of the ketone to the aldehyde is typically in the range 1:4 to 10:1, preferably 5:1 to 2:1 and most preferably 3:1 to 4.5:1. The amount of water in the mixture is generally from 0.1 to 2 parts, most preferably about 0.3 to 0.8 parts, by weight per part by weight organic components in the mixture. The alkali is preferably sodium hydroxide. The amount of phase transfer catalyst agent is generally in the range 0.01 to 1%, preferably 0.05 to 0.5%, by weight of the reaction mixture.

The reaction mixture is generally formed by adding the aldehyde to a preformed mixture of the other components. Reaction is generally conducted at 50 to 95°C, preferably 75 to 85°C, over a period of 1 to 6 hours, generally 3 to 4 hours.

This reaction results in the production of a saturated intermediate, 2,7 - dimethyl - oct - 6 - hydroxy - 4 - one. The saturated intermediate must then be dehydrated to form the devised compound. Dehydration can be conducted on the saturated compound after isolation from the reaction mixture, for instance by decanting off the aqueous layer and washing the organic layer, containing the saturated compounds, free of alkali with water, but generally it is satisfactory and is preferred to conduct the dehydration on the reaction mixture without isolation of the saturated intermediate.

Dehydration can be effected by distilling in the presence of a dehydrating agent such as paratoluene-sulphonic acid. However, we find that the use of a strong acid promotes the formation of the 6-ene and/or 7-ene compounds and that improved yield of the 5-ene compound is achieved with weaker acids. Suitable acids that are weaker than paratoluene sulphonic acid and which are useful in the invention include aliphatic carboxylic acids, typically saturated aliphatic acids containing up to 6 carbon atoms, and most preferably aliphatic dicarboxylic acids, oxalic acid and maleic acid being particularly preferred. The acid preferably has a dissociation constant of from 1 to 2.5.

The preferred way of dehydrating is to heat the reaction mixture under reflux in the presence of the selected acid. The amount of acid is often in the range 0.1 to 5% (by weight based on the weight of saturated intermediate) with amounts of 0.5 to 5% being particularly preferred when using oxalic acid and other saturated aliphatic acids.

The distillation is preferably effected by heating the reaction mixture under reflux, generally at atmospheric pressure, for instance for from 4 to 8 hours and azeotropically removing the water.

The following are examples of the invention.

Example 1

To a mixture of methyl isobutyl ketone (1014 gms) water (690 mls) sodium hydroxide pellets (7 gms) and cetyltrimethylammonium bromide (3.6 gms) stirred at 80°C is added isobutyraldehyde (480 gms) over a period of one hour. After the addition is complete, the reaction is stirred at 80°C for a further four hours. The stirring is then stopped and the aqueous layer is decanted off and rejected. The organic layer containing the desired intermediate is then washed free of alkali with water.

Oxalic acid (9 gms) is then added to washed product containing the intermediate and the mixture is refluxed, removing the water formed via a Dean and Stark head. When no more water is obtained, the mixture is cooled and washed free of acid with aqueous sodium carbonate solution.

Fractionation of the product yields ca. 400 gms of a product having a boiling point 69°C at 5 torr. Analysis by gas liquid chromatography indicates a composition of 80% dimethyloct - 5 - enone and 20% dimethyloct - 6 - enone.

Example 2

Repeating Example 1 by heating (in the dehydration step) with 2 gms of p-toluene sulphonic acid for 14 hours yields a product whose composition is 23% dimethyloct - 5 - enone, 66% dimethyloct - 6 - enone and 11% dimethyloct - 7 - enone. The odour properties of this mixture are less effective and useful than those of the mixture of Example 1.

Example 3

A perfumery concentrate is formulated in conventional manner from:

| | |
|---|---|
| Musk ketone | 0.23 |
| Dipropylene Glycol | 0.57 |
| Citral pure | 0.75 |
| Hedione | 0.60 |
| Cis 3 hexenyl salicylate | 0.08 |
| Ethoxy hexoxy ethane (lilivert) | 1.50 |
| Mandarin aldehyde 10 | 0.02 |
| Neoroli Synth. BB40 | 1.28 |
| Orange oil sweet Florida | 24.06 |
| Osyrol | 4.51 |
| Patchouli oil Iron free | 0.23 |
| Rosemary oil Spanish | 0.60 |
| Spearmint oil American | 0.15 |
| Thyme oil White Rect. | 0.23 |
| Vertofix Cobur | 0.53 |
| Bergamont Synth. 6749 | 63.16 |
| Blend of Example 1 | 1.50 |
| | 100.00 |

The process of Example 1 is a very simple process to operate and gives a high yield of the defined blend at relatively lost cost. The perfumery properties of the concentrate of Example 3, and of other compositions containing the 5-ene compound are very desirable. These

concentrates can be used in the preparation of consumer compositions of the types discussed above.

**Claims**

1. The use of the compound 2,7 - dimethyl - oct - 5 - ene - 4 - one as a perfumery component in a perfumery composition containing at least one odiferous compounds that is not a dimethyl octenone.

2. Ths use of the compound 2,7 - dimethyl - oct - 5 - ene - 4 - one as a perfumery component in a perfumery composition containing at least one odiferous compound that is not a dimethyl octenone and wherein the 2,7 - dimethyl - oct - 5 - ene - 4 - one is made by reacting 2 - methyl - pentan - 4 - one with isobutyraldehyde and dehydrating the resultant saturated product.

3. A process for making a perfumery composition comprising making 2,7 - dimethyl - oct - 5 - ene - 4 - one by reacting 2 - methyl - pentan - 4 - one with isobutyraldehyde and dehydrating the resultant saturated product and then combining the resultant 2,7 - dimethyl - oct - 5 - ene - 4 - one with at least one odiferous compound that is not a dimethyl octenone.

**Patentansprüche**

1. Verwendung der Verbindung 2,7 - Dimethyl - oct - 5 - en - 4 - on als Riechstoff in einer Riechstoffzusammensetzung, die mindestens eine wohlriechende Verbindung enthält, die kein Dimethyloctenon ist.

2. Verwendung der Verbindung 2,7 - Dimethyl - oct - 5 - en - 4 - on als Riechstoff in einer Riechstoffzusammensetzung, die mindestens eine wohlriechende Verbindung enthält, die kein Dimethyloctenon ist, und wobei das 2,7 - Dimethyl - oct - 5 - en - 4 - on durch Reaktion von 2 - Methyl - pentan - 4 - on mit Isobutyraldehyd und Dehydratisierung des entstehenden gesättigten Produktes hergestellt wird.

3. Verfahren zur Herstellung einer Riechstoffzusammensetzung, welches die Herstellung von 2,7 - Dimethyl - oct - 5 - en - 4 - on durch Reaktion von 2 - Methyl - pentan - 4 - on mit Isobutyraldehyd und Dehydratisierung des entstehenden gesättigten Produktes und daran anschließend die Kombinierung des erhaltenen 2,7 - Dimethyl - oct - 5 - en - 4 - ons mit mindestens einer wohlriechenden Verbindung, die kein Dimethyloctenon ist, umfaßt.

**Revendications**

1. L'utilisation du composé 2,7 - diméthyl - 5 - octène - 4 - one comme composant de parfumerie dans une composition de parfumerie contenant au moins un composé odorant qui n'est pas un diméthylocténone.

2. L'utilisation du composé 2,7 - diméthyl - 5 - octène - 4 - one comme composant de parfumerie dans une composition de parfumerie contenant au moins un composé odorant qui n'est pas un diméthylocténone et dans laquelle la 2,7 - diméthyl - 5 - octène - 4 - one est fabriquée en faisant réagir la 2 - méthyl - pentane - 4 - one avec l'isobutyraldéhyde et en déshydratant le produit saturé résultant.

3. Un procédé pour fabriquer une composition de parfumerie consistant à préparer la 2,7 - diméthyl - 5 - octène - 4 - one en faisant réagir le 2 - méthyl - pentane - 4 - one avec l'isobutyraldéhyde et en déshydratant le produit saturé résultant et ensuite à combiner la 2,7 - diméthyl - 5 - octène - 4 - one avec au moins un composé odorant qui n'est pas une diméthylocténone.